# EUROPEAN PATENT SPECIFICATION

(11) **EP 0 512 419 B1**
(45) Date of publication and mention of the grant of the patent: **14.12.1994**
(21) Application number: 92107402.7
(22) Date of filing: 04.05.1992
(51) Int. Cl.: C07D 243/02, A61K 31/55

(54) **Process for the preparation of high-purity 1-[3'-(chloro)-phenyl]-4-[methyl]-7,8-di-[methoxy]-5H-2,3-benzo diazepine**
Verfahren zur Herstellung von 1-[3'-(chloro)-phenyl]-4-[methyl]-7,8-di-[methoxy]-5H-2,3-Benzodiazepin hoher Reinheit
Procédé pour la préparation de 1-[3'-(chloro)-phényl]-4-[méthyl]-7,8-di-[méthoxy]-5H-2,3-benzodiazépine de haute pureté

(30) Priority: 03.05.1991 HU 148291
(43) Date of publication of application: 11.11.1992
(73) Proprietor: EGIS GYOGYSZERGYAR, H-1106 Budapest (HU)
(72) Inventor: Somogyi, György, H-1148 Budapest (HU); Botka, Péter, H-1033 Budapest (HU); Hámori, Tamás, Dr., H-1149 Budapest (HU); Körösi, Jenó, Dr., H-1013 Budapest (HU); Kiss, Csilla, H-1035 Budapest (HU); Bidlo née Iglói, Mária, Dr., H-1113 Budapest (HU); Balogh, Tibor, Dr., H-1138 Budapest (HU); Horváth, Gyula, Dr., H-1052 Budapest (HU); Simay, Antal, Dr., H-1124 Budapest (HU); Gyenge, Rózsa, Dr., H-1013 Budapest (HU); Moravcsik, Imre, H-1095 Budapest (HU); Orbán, Ernö, Dr., H-1119 Budapest (HU); Uskert née Dievald, Emilia, Dr., H-1117 Budapest (HU)
(74) Representative: Beszédes, Stephan G., Dr.

(56) References cited:
- FR-A- 2 566 774

## Description

This invention relates to a process for the preparation of 1-[3'-(chloro)-phenyl]-4-[methyl]-7,8-di-[methoxy]-5H-2,3-benzodiazepine of formula 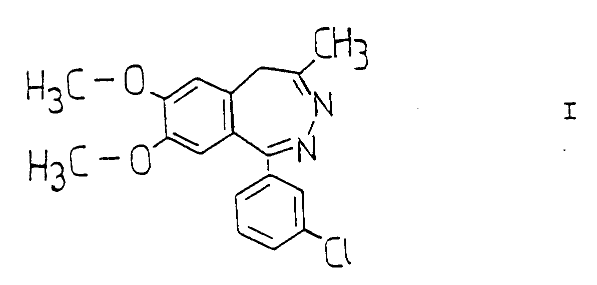
in high purity and in a quality suitable for pharmaceutical purposes.

In the present patent application 1-[3'-(chloro)-phenyl]-4-[methyl]-7,8-di-[methoxy]-5H-2,3-benzodiazepine is considered to be of high purity if - according to high pressure liquid chromatographic examinations - the total contamination content thereof does not exceed 0.3% by weight.

The 5H-2,3-benzodiazepine derivatives possess generally valuable central nervous system activity, such as antiaggressive, antidepressant and narcotizing effects. According to preclinical and clinical trials one of them, the 1-[3'-(chloro)-phenyl]-4-[methyl]-7,8-di-[methoxy]-derivative is to be considered as a drug candidate (Hungarian patent specifications Nos. 179,018 and 191,702).

Several methods are known in the art for the preparation of 5H-2,3-benzodiazepines. Among them the process disclosed by FR-A-2 566 774 (= Hungarian patent specification No. 191,702) seems to be the most favourable one considering either the high yield or the feasibility on industrial scale. According to this process 1-[3'-(chloro)-phenyl]-4-[methyl]-7,8-di-[methoxy]-5H-2,3-benzodiazepine of formula I is produced by reacting 2-(acetonyl)-3'-(chloro)-4,5-di-(methoxy)-benzophenone of formula 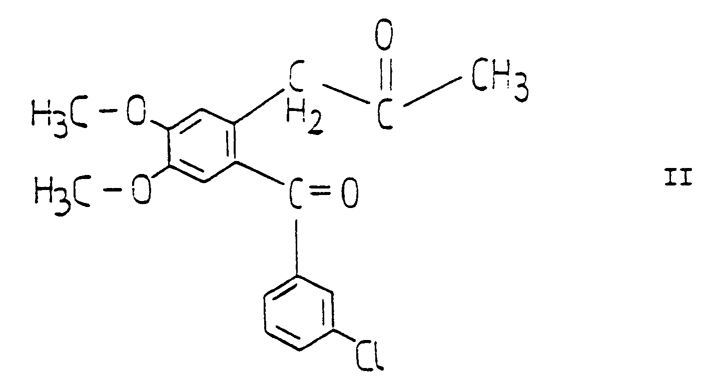
with 3 to 7 moles of hydrazine hydrate, calculated on 1 mole of 2-(acetonyl)-3'-(chloro)-4,5-di-(methoxy)-benzophenone of formula II, at a temperature from -20°C to +110°C. The reaction time depends on the temperature and varies from 1 to 168 hour(s). The yield of the crude product isolated from the reaction mixture amounts to 80 to 85% and the efficiency of recrystallization of the crude product from isopropanol is about 90%.

According to own investigations the 1-[3'-(chloro)-phenyl]-4-[methyl]-7,8-di-[methoxy]-5H-2,3-benzodiazepine of formula I thus obtained contains 3 characteristic contaminations. Semiquantitive thin layer chromatographic examinations show [adsorbent: silica gel HF₂₅₄ , eluent: a 2 : 8 (by volume) mixture of benzene and ethyl acetate, evaluation in UV light] that the total amount thereof is about 1% by weight. The contaminating components were separated by preparative layer chromatography and identified by spectroscopic methods (IR, NMR, MS).

Said contaminations proved to be the following compounds:
a) 1-[3'-(Chloro)-phenyl]-4-[methyl]-5-[hydroxy]-7,8-di-[methoxy]-5H-2,3-benzodiazepine of formula 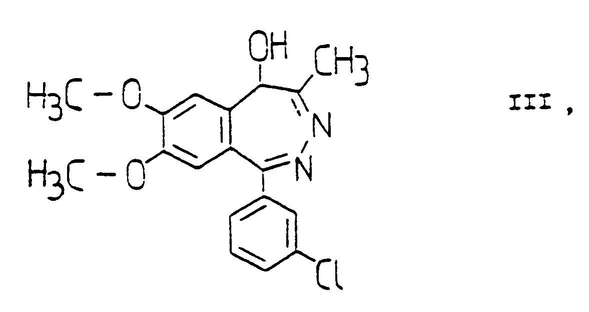 melting point: 181 to 184°C (from isopropanol).
b) 1-[3'-(Chloro)-phenyl]-4-[methyl]-8-[methoxy]-5H-2,3-benzodiazepine of formula 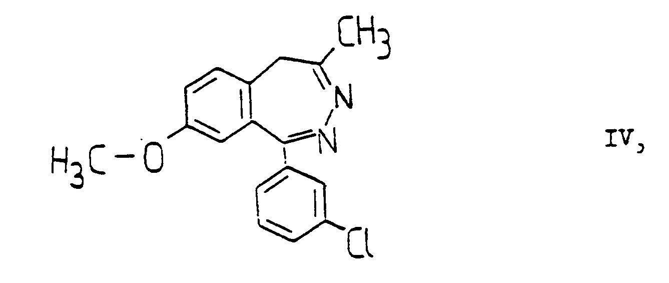 melting point: 128 to 129°C (from isopropanol).
c) 1-[3'-(Chloro)-phenyl]-3-[methyl]-6,7-di-[methoxy]-isoquinolinone of formula 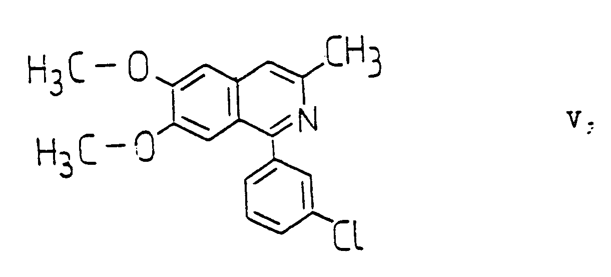 melting point: 163 to 165°C (from absolute ethanol).

High pressure liquid chromatographic examinations showed that the recrystallized 1-[3'-(chloro)-phenyl]-4-[methyl]-7,8-di-[methoxy]-5H-2,3-benzodiazepine of formula I generally contained 0.1 to 0.3% by weight of 1-[3'-(chloro)-phenyl]-4-[methyl]-8-[methoxy]-5H-2,3-benzodiazepine of formula IV and 1-[3'-(chloro)-phenyl]-3-[methyl]-6,7-di-[methoxy]-isoquinolinone of formula V each. The 1-[3'-(chloro)-phenyl]-4-[methyl]-5-[hydroxy]-7,8-di-[methoxy]-5H-2,3-benzodiazepine of formula III can be detected generally in an amount of 0.5 to 1.0% by weight in the crude product obtained according to the method described in FR-A-2 566 774, but in certain cases - even when using the same starting substances and applying apparently the same reaction conditions - the amount thereof may be surprisingly as high as 2 to 3% by weight.

The crude product containing such a high amount of contaminations cannot be purified by the usual recrystallization. In own experiments a great number of solvents and solvent mixtures [such as alcohols comprising 1 to 3 carbon atom(s), acetic acid esters, aliphatic ketones, tetrahydrofuran, benzene, mixtures thereof and the mixture of these solvents formed with dimethylformamide, chlorinated hydrocarbons, water and acetic acid] have been tried. According to these experiments the 1-[3'-(chloro)-phenyl]-4-[methyl]-5-[hydroxy]-7,8-di-[methoxy]-5H-2,3-benzodiazepine contamination of formula III practically separates together with the 1-[3'-(chloro)-phenyl]-4-[methyl]-7,8-di-[methoxy]-5H-2,3-benzodiazepine of formula I.

When the product containing 2% by weight of 1-[3'-(chloro)-phenyl]-4-[methyl]-5-[hydroxy]-7,8-di-[methoxy]-5H-2,3-benzodiazepine of formula III is purified by fractional crystallization and recrystallized from a 30-fold volume of ethyl acetate related to the weight of the product, a 1st fraction of 52,4% yield containing 3% by weight of 1-[3'-(chloro)-phenyl]-4-[methyl]-5-[hydroxy]-7,8-di-[methoxy]-5H-2,3-benzodiazepine of formula III and a 2nd fraction of 40.6% yield containing 0.6% by weight of 1-[3'-(chloro)-phenyl]-4-[methyl]-5-[hydroxy]-7,8-di-[methoxy]-5H-2,3-benzodiazepine of formula III are obtained. In order to decrease the 1-[3'-(chloro)-phenyl]-4-[methyl]-5-[hydroxy]-7,8-di-[methoxy]-5H-2,3-benzodiazepine content at least below 0.5% by weight, fraction 2 is to be subjected again to fractional crystallization. This step, however, results in an unacceptably high loss of substance.

Thus the problem underlying the invention is to create a simple and more effective method for the preparation and/or purification of 1-[3'-(chloro)-phenyl]-4-[methyl]-7,8-di-[methoxy]-5H-2,3-benzodiazepine to yield this in a high purity and quality suitable for pharmaceutical purposes.

The above problem surprisingly has been solved by the invention.

Surprisingly it has been found that by using special conditions in the process starting from the 2-(acetonyl)-3'-(chloro)-4,5-di-(methoxy)-benzophenone of formula II and hydrazine hydrate the formation of contaminations of formulae III to V during the process can be eliminated or considerably reduced.

The invention is based on the surprising recognition that if the reaction of the 2-(acetonyl)-3'-(chloro)-4,5-di-(methoxy)-benzophenone of formula II with the hydrazine hydrate is carried out in the absence of air oxygen, practically no 1-[3'-(chloro)-phenyl]-4-[methyl]-5-[hydroxy]-7,8-di-[methoxy]-5H-2,3-benzodiazepine of formula III is formed in the reaction. It was not at all aforeseen that while hydrazine hydrate being present in the reaction mixture in a large excess - although it is a strong reducing agent - cannot prevent the oxydation side-reactions leading to the formation of 1-[3'-(chloro)-phenyl]-4-[methyl]-5-[hydroxy]-7,8-di-[methoxy]-5H-2,3-benzodiazepine of formula III, the formation of this compound can completely be prevented in the absence of air oxygen. Besides, it has been found that by choosing appropriate reaction conditions - such as temperature or solvent - the formation of the further contaminating compounds of formulae IV and V can be prevented, too.

Thus, according to an aspect of the present invention there is provided for a process for the preparation of 1-[3'-(chloro)-phenyl]-4-[methyl]-7,8-di-[methoxy]-5H-2,3-benzodiazepine of formula 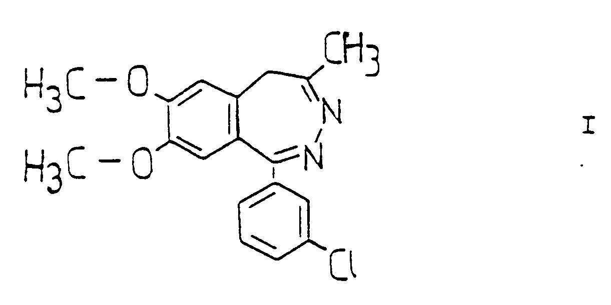
the total contamination content thereof not exceeding 0,3% by weight - according to high liquid pressure chromatographic examinations - by reacting 1 mole of 2-(acetonyl)-3'-(chloro)-4,5-di-(methoxy)-benzophenone of formula 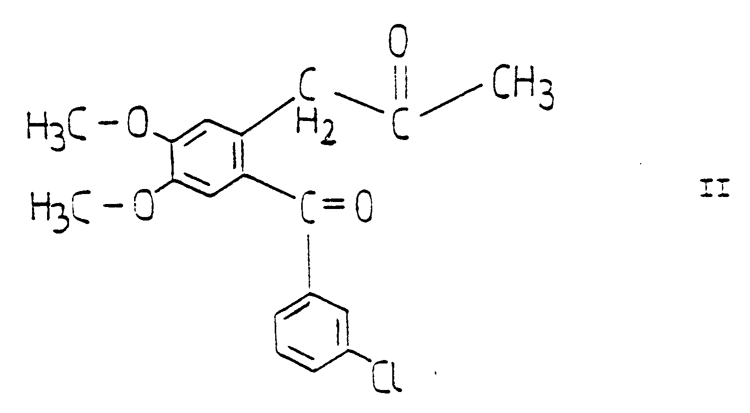
with 3 to 7 moles of hydrazine hydrate in an organic solvent or in a mixture of organic solvents, at a temperature from 15°C to 85°C, and subsequently recrystallizing the crude product from an aliphatic alcohol containing 1 to 5 carbon atom(s), which is characterized in that the reaction of the 2-(acetonyl)-3'-(chloro)-4,5-di-(methoxy)-benzophenone with the hydrazine hydrate is carried out in the absence of air oxygen.

Optionally the above reaction is carried out in the presence of water.

The starting 2-(acetonyl)-3'-(chloro)-4,5-di-(methoxy)-benzophenone of formula II can be prepared as described in Hungarian patent specification No. 194,529.

Preferably as solvent(s) 1 or more monovalent or polyvalent aliphatic alcohol(s) comprising 1 to 5 carbon atom(s), dimethylformamide, dimethyl sulfoxide, 1 or more chlorinated hydrocarbon(s), peroxide-free dioxane or tetrahydrofuran or mixtures thereof or - in case of water-miscible solvents - a mixture of these solvents with water is/are used.

It is preferable - but not necessary - to deoxygenate the solvent by simple boiling before it is used. The reaction vessel can be deoxygenated by known methods, e.g. by evacuating or flushing the air space of the equipment with an inert gas. For this purpose preferably nitrogen and/or a noble gas is used. Thus it is preferred to carry out the reaction under an atmosphere of nitrogen and/or a noble gas.

Preferably as alcohol(s) for the recrystallization of the crude 1-[3'-(chloro)-phenyl]-4-[methyl]-7,8-di-[methoxy]-5H-2,3-benzodiazepine product such having 2 and/or 3 carbon atoms, particularly ethanol and/or isopropanol, is/are used. The preferred quantities of them are 10 to 12, particularly 12, parts by volume calculated on the weight of the crude 1-[3'-(chloro)-phenyl]-4-[methyl]-7,8-di-[methoxy]-5H-2,3-benzodiazepine.

According to a preferred embodiment this process of the invention is carried out as follows. The 2-(acetonyl)-3'-(chloro)-4,5-di-(methoxy)-benzophenone of formula II is suspended in an aliphatic alcohol comprising 1 to 5 carbon atom(s), optionally containing water, the vessel is deoxygenated with nitrogen or argon and 98 to 100% by weight hydrazine hydrate is added to the suspension while stirring. Then the inert gas atmosphere is maintained and the mixture is reacted at a temperature from 25°C to 70°C. The reaction time - considering the extreme temperature values - lasts for about 78 and 3 hours, respectively. The reaction mixture is then optionally diluted with distilled water and the excess of hydrazine hydrate is neutralized with acetic acid. The crystalline product separating upon cooling is filtered, washed with an aqueous alcohol and dried. The yield of the crude product amounts to 85 to 90%. The crude product is recrystallized from 10 to 12 parts by volume of ethanol or isopropanol - related to the weight of the former -. The solution is optionally treated with charcoal.

According to the invention it is also possible to effectively purify the 1-[3'-(chloro)-phenyl]-4-[methyl]-7,8-di-[methoxy]-5H-2,3-benzodiazepine of formula I contaminated due to the defects in the effectuation of the process for the preparation of a high-purity product starting from the 2-(acetonyl)-3'-(chloro)-4,5-di-(methoxy)-benzophenone of formula II and/or owing to the accidental contaminations of the starting 2-(acetonyl)-3'-(chloro)-4,5-di-(methoxy)-benzophenone of formula II. In this regard the invention is based on the surprising recognition that the hydroxy group of 1-[3'-(chloro)-phenyl]-4-[methyl]-5-[hydroxy]-7,8-di-[methoxy]-5H-2,3-benzodiazepine of formula III - although it is not attached to an aromatic ring - has under certain conditions a phenolic character. In case of the process according to the invention this means that if 1-[3'-(chloro)-phenyl]-4-[methyl]-7,8-di-[methoxy]-5H-2,3-benzodiazepine of formula I contaminated with 1-[3'-(chloro)-phenyl]-4-[methyl]-5-[hydroxy]-7,8-di-[methoxy]-5H-2,3-benzodiazepine of formula III is dissolved in an solvent and treated with an alkali hydroxide, alkali carbonate or alkali alcoholate or the dissolution of these two groups of compounds is made in the inverse order or simultaneously and then the 1-[3'-(chloro)-phenyl]-4-[methyl]-7,8-di-[methoxy]-5H-2,3-benzodiazepine of formula I is crystallized from the solution, this latter compound separates in a high purity and yield, while the corresponding alkali derivative of the 1-[3'-(chloro)-phenyl]-4-[methyl]-5-[hydroxy]-7,8-di-[methoxy]-5H-2,3-benzodiazepine of formula III, owing to its better solubility, remains dissolved. The recrystallization in this process is an effective method for the purification of the desired 1-[3'-(chloro)-phenyl]-4-[methyl]-7,8-di-[methoxy]-5H-2,3-benzodiazepine of formula I additionally contaminated with 1-[3'-(chloro)-phenyl]-3-[methyl]-6,7-di-[methoxy]-isoquinolinone of formula V, too.

Thus, according to a further aspect of the present invention there is provided for a process for preparing purified 1-[3'-(chloro)-phenyl]-4-[methyl]-7,8-di-[methoxy]-5H-2,3-benzodiazepine of formula I from such containing contamination(s) of formula 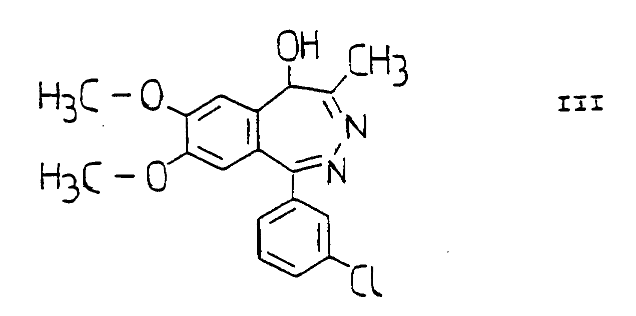
and optionally of formula 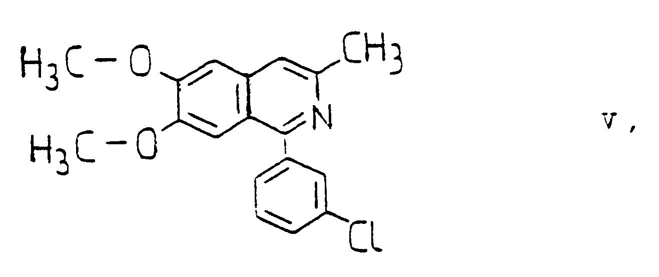
optionally prepared according to the first-named process according to the invention, which is characterized in that first the contaminated 1-[3'-(chloro)-phenyl]-4-[methyl]-7,8-di-[methoxy]-5H-2,3-benzodiazepine of formula I is treated with a solvent and an alkali hydroxide, alkali carbonate or alkali alcoholate the solvent being such in which at least a 0.005 mole/l solution from the said alkali compounds can be prepared and thereafter the compound 1-[3'-(chloro)-phenyl]-4-[methyl]-7,8-di-[methoxy]-5H-2,3-benzodiazepine of formula I is crystallized from the obtained solution.

In other terms first the contaminated 1-[3'-(chloro)-phenyl]-4-[methyl]-7,8-di-[methoxy]-5H-2,3-benzodiazepine of formula I and/or an alkali hydroxyde, alcali carbonate or alkali alcoholate is/are dissolved in a solvent and in case there have not been dissolved simultaneously both of them then also that not dissolved in advance is dissolved in the solvent and thereafter the compound 1-[3'-(chloro)-phenyl]-4-[methyl]-7,8-di-[methoxy]-5H-2,3-benzodiazepine of formula I is crystallized from the solution. The preferred variant is to dissolve first the alkali compound and successively the 1-[3'-(chloro)-phenyl]-4-[methyl]-7,8-di-[methoxy]-5H-2,3-benzodiazepine of formula I.

Optionally the crystallized 1-[3'-(chloro)-phenyl]-4-[methyl]-7,8-di-[methoxy]-5H-2,3-benzodiazepine of formula I obtained is recrystallized from an aliphatic alcohol containing 1 to 5 carbon atom(s). Any solvent or solvent mixture suitable for the recrystallization of the 1-[3'-(chloro)-phenyl]-4-[methyl]-7,8-di-[methoxy]-5H-2,3-benzodiazepine of formula I and inert towards the applied alkali hydroxide, carbonate or alcoholate can be used for the reaction, provided that at least a 0.005 mole/l solution can be prepared from the said alkali compounds in the said solvent. It is preferable to use straight or branched chained mono- or polyvalent aliphatic alcohols comprising 1 to 5, particularly 3 or 4, carbon atom(s) or mixtures thereof, most particularly isopropanol and/or tert.-butanol, as solvent. The solvents are not required to be anhydrous, but on increasing the water content the effectiveness of the purifying process is decreasing. (Even on applying a solvent containing 10% by weight of water for the recrystallization a significant improvement of quality can be achieved.)

Preferably from 10 to 20 parts by volume of the aliphatic alcohol(s) calculated on the weight of the 1-[3'-(chloro)-phenyl]-4-[methyl]-7,8-di-[methoxy]-5H-2,3-benzodiazepine of formula I are used in case of such having 3 carbon atoms 10 to 14 parts, above all 12 parts, by volume and in case of such having 4 carbon atoms 16 to 20 parts, above all 18 parts, by volume being particularly preferred.

Preferably the treatment with the alkali hydroxide, alkali carbonate or alkali alcoholate is carried out at the boiling point of the solvent.

As alkali hydroxides and carbonates advantageously the easily available sodium or potassium compounds can be used. Preferably as alkali alcoholates sodium or potassium alcoholates prepared from straight or branched chained alcohols comprising 1 to 4 carbon atom(s) are applied. They can be obtained either from the commerce or prepared in situ from an alcohol and alkali metal. The upper limit of the amount of the alkali compounds used for the reaction is restricted by the solubility thereof in the solvent in question, but suitably at least 1 mole of alkali compound is to be used for 1 mole of 1-[3'-(chloro)-phenyl]-4-[methyl]-5-[hydroxy]-7,8-di-[methoxy]-5H-2,3-benzodiazepine contamination of formula III. (Using more than 35 moles of alkali compound does not imply advantages.)

Preferably as aliphatic alcohol(s) for the recrystallization such having 2 or 3 carbon atoms, particularly ethanol and/or isopropanol, is/are used. The preferred quantities are 10 to 14, particularly 12, parts by volume calculated on the 1-[3'-(chloro)-phenyl]-4-[methyl]-7,8-di-[methoxy]-5H-2,3-benzodiazepine product of formula I.

According to a preferred embodiment of the purifying process of the invention it is worked as follows. 5 to 30 Moles of an alkali hydroxide, alkali carbonate or alkali alcoholate related to 1 mole of 1-[3'-(chloro)-phenyl]-4-[methyl]-5-[hydroxy]-7,8-di-[methoxy]-5H-2,3-benzodiazepine are added to a 12-fold volume of isopropanol or 18-fold volume of tert.-butanol related to the weight of the 1-[3'-(chloro)-phenyl]-4-[methyl]-7,8-di-[methoxy]-5H-2,3-benzodiazepine of formula I containing 1 to 3% by weight of 1-[3'-(chloro)-phenyl]-4-[methyl]-5-[hydroxy]-7,8-di-[methoxy]-5H-2,3-benzodiazepine of formula III, the solution thus obtained is heated to boiling and the crude 1-[3'-(chloro)-phenyl]-4-[methyl]-7,8-di-[methoxy]-5H-2,3-benzodiazepine of formula I to be purified is dissolved in it. From the solution thus obtained the 1-[3'-(chloro)-phenyl]-4-[methyl]-7,8-di-[methoxy]-5H-2,3-benzodiazepine of formula I is crystallized by cooling, the product is filtered, washed successively with isopropanol or tert.-butanol and distilled water until neutral and dried. Thus, the contamination content of the substance is decreased by about one order of magnitude, and the yield of the process amounts to 88 to 95%. For pharmaceutical use the product is preferably recrystallized again from a 12-fold volume of isopropanol or more preferably of ethanol. This recrystallization can be performed with a yield of 94 to 97%. According to HPLC examinations the product thus obtained contains not more than 0.3% of contaminations, generally 1-[3'-(chloro)-phenyl]-4-[methyl]-5-[hydroxy]-7,8-di-[methoxy]-5H-2,3-benzodiazepine of formula III and 1-[3'-(chloro)-phenyl]-4-[methyl]-8-[methoxy]-5H-2,3-benzodiazepine of formula IV.

The invention renders possible the preparation of 1-[3'-(chloro)-phenyl]-4-[methyl]-7,8-di-[methoxy]-5H-2,3-benzodiazepine containing not more than 0.3% of contamination. This result is achieved by a simple measure, that is by carrying out the reaction in the absence of air oxygen. Said measure, while it results in a considerable improvement in the quality of the product, do not cause losses in the yield compared with the known processes, it even makes possible the stabilization of the yield at a high level. The purification process according to the invention represents a simple and effective method for the purification of 1-[3'-(chloro)-phenyl]-4-[methyl]-7,8-di-[methoxy]-5H-2,3-benzodiazepine contaminated for any reason, containing especially 1-[3'-(chloro)-phenyl]-4-[methyl]-5-[hydroxy]-7,8-di-[methoxy]-5H-2,3-benzodiazepine of formula III.

Carrying out the invention is economically feasible on industrial scale, too, and the product prepared or purified thereby is suitable for pharmaceutical use based on its purity grade of 99.7 to 100.0%.

The product thus obtained can be converted directly into a pharmaceutical composition, preferably subsequent to a micronization ensuring a mean particle size below 25 µm.

The invention is further illustrated by the following Examples.

### Example 1

### 1-[3'-(Chloro)-phenyl]-4-[methyl]-7,8-di-[methoxy]-5H-2,3-benzodiazepine

20.0 g (0.060 mole) of 2-(acetonyl)-3'-(chloro)-4,5-di-(methoxy)-benzophenone (II) are added to 70 ml of isopropanol and air is expelled from the flask with a nitrogen stream. Then 18.0 ml (0.367 mole) of 98 to 100% by weight hydrazine hydrate are added within 1 to 2 minutes, under slow stirring, and while a nitrogen atmosphere is maintained in the vessel the reaction mixture is stirred at 28 to 32°C for 78 hours. It is cooled to 20°C, diluted with 70 ml of distilled water, then 17.1 ml of glacial acetic acid are added. A crystalline product gets separated from the orange-red solution within 1 to 2 minutes. The crystalline suspension is stirred at 0 to 5°C for 4 hours, filtered, washed with a 1 : 1 (by volume) mixture of isopropanol and water and dried at 40°C. Thus 17.85 g of crude product are obtained. M.p.: 168.5 to 170°C. The crude product is dissolved in 214 ml of boiling isopropanol, the solution is treated with charcoal and cooled to 0 to 5°C under stirring. The separated product is filtered off, washed with isopropanol of 0 to 5°C and dried at 40°C. Thus 16.1 g (80.2% by weight, related to the 2-(acetonyl)-3'-(chloro)-4,5-di-(methoxy)-benzophenone of formula II of the desired compound are obtained in the form of white crystals which, according to HPLC examination, do not contain a measurable amount of contamination. The product is suitable for pharmaceutical purposes either in this form or - if desired - subsequent to a micronization resulting in an average particle size of below 25 µm.
M.p.: 168 to 169°C.

### Example 2

### 1-[3'-(Chloro)-phenyl]-4-[methyl]-7,8-di-[methoxy]-5H-2,3-benzodiazepine

20.0 g (0.060 mole) of 2-(acetonyl)-3'-(chloro)-4,5-di-(methoxy)-benzophenone (II) are portionwise added to 100 ml of a 9 : 1 (by volume) mixture of methanol and water, and air is expelled from the flask with an argon stream. Then 17.6 ml (0.363 mole) of 98 to 100% by weight hydrazine hydrate are added under slow stirring, within 1 to 2 minutes, and while an argon atmosphere is maintained in the vessel the reaction mixture is boiled (68°C) for 3 hours. It is cooled to 20°C and 16.5 ml of glacial acetic acid are added. The crystalline suspension thus obtained is diluted with 80 ml of distilled water and stirred at 0 to 5°C for 4 hours. The separated product is filtered off, washed with a 1 : 1 (by volume) mixture of methanol and distilled water and dried at 40°C. Thus 18.0 g of crude product are obtained. M.p.: 166 to 169°C. The crude product is dissolved in 216 ml of boiling abs. ethanol, the solution is treated with charcoal, washed with ethanol of 0 to 5°C and dried at 40°C. Thus 15.4 g (78.1% by weight, related to the 2-(acetonyl)-3'-(chloro)-4,5-di-(methoxy)-benzophenone of formula II) of the desired compound are obtained, which - according to HPLC examination - does not contain any contamination. M.p.: 168 to 169°C.

### Example 3

### 1-[3'-(Chloro)-phenyl]-4-[methyl]-7,8-di-[methoxy]-5H-2,3-benzodiazepine

One proceeds as described in Example 2 except that the crude product is recrystallized from 216 ml of isopropanol. Thus 16.43 g (83.1% by weight, related to the 2-(acetonyl)-3'-(chloro)-4,5-di-(methoxy)-benzophenone of formula II) of 1-[3'-(chloro)-phenyl]-4-[methyl]-7,8-di-[methoxy]-5H-2,3-benzodiazepine are obtained. According to HPLC examinations the product contains 0.12% by weight of 1-[3'-(chloro)-phenyl]-4-[methyl]-8-[methoxy]-5H-2,3-benzodiazepine of formula IV.
M.p.: 168 to 170°C.

### Example 4

### 1-[3'-(Chloro)-phenyl]-4-[methyl]-7,8-di-[methoxy]-5H-2,3-benzodiazepine from contaminated product by purification

50.0 g (0.15 mole) of 1-[3'-(chloro)-phenyl]-4-[methyl]-7,8-di-[methoxy]-5H-2,3-benzodiazepine (I) containing 1,4% by weight of 1-[3'-(chloro)-phenyl]-4-[methyl]-5-[hydroxy]-7,8-di-[methoxy]-5H-2,3-benzodiazepine contamination of formula III according to HPLC examinations are dissolved in 600 ml of isopropanol containing potassium hydroxide in an amount of 0.39 g/100 ml. When the dissolution is complete, the mixture is cooled to 0 to 5°C and allowed to crystallize at the same temperature for 4 hours. The separated crystals are filtered off, washed until neutral with isopropanol of 0 to 5°C and distilled water and dried to constant weight at 40°C. Thus 47.6 g of the desired compound are obtained which are recrystallized from a 12-fold amount by volume of isopropanol. Yield: 46.2 g (92.4% by weight). According to HPLC examinations the product contains 0.20% by weight of 1-[3'-(chloro)-phenyl]-4-[methyl]-5-[hydroxy]-7,8-di-[methoxy]-5H-2,3-benzodiazepine contamination of formula III.
M.p.: 168 to 169°C.

### Example 5

### 1-[3'-(chloro)-phenyl]-4-[methyl]-7,8-di-[methoxy]-5H-2,3-benzodiazepine from contaminated product by purification

0.15 g (1.3 mmole) of potassium tert.-butylate are added to 90 ml of tert.-butanol, and 5.0 g (15 mmoles) of 1-[3'-(chloro)-phenyl]-4-[methyl]-7,8-di-[methoxy]-5H-2,3-benzodiazepine of formula I containing - according to HPLC examinations - 1.5% by weight (0.22 mmoles) of 1-[3'-(chloro)-phenyl]-4-[methyl]-5-[hydroxy]-7,8-di-[methoxy]-5H-2,3-benzodiazepine contamination of formula III are dissolved in the mixture at the boiling point thereof. Then it is cooled to 25 to 27°C and allowed to crystallize at the same temperature for 4 hours. The separated crystals are filtered off and washed to neutral with tert.-butanol of 25 to 27°C and with distilled water, then they are dried to constant weight at 40°C. Thus 4.4 g of white crystalline product are obtained which are recrystallized from a 12-fold volume of ethanol related to the weight of the product to yield 4.2 g (84.0% by weight) of the desired compound containing - according to HPLC examinations - 0.12% by weight of 1-[3'-(chloro)-phenyl]-4-[methyl]-5-[hydroxy]-7,8-di-[methoxy]-5H-2,3-benzodiazepine contamination of formula III.
M.p.: 167 to 167.5°C.

### Example 6

### 1-[3'-(Chloro)-phenyl]-4-[methyl]-7,8-di-[methoxy]-5H-2,3-benzodiazepine from contaminated product by purification

0.5 g (5.0 mmoles) of sodium ethylate are dissolved in 70 ml of isopropanol, then 5.0 g (15 mmoles) of 1-[3'-(chloro)-phenyl]-4-[methyl]-7,8-di-[methoxy]-5H-2,3-benzodiazepine of formula I containing - according to HPLC examinations - 1.5% by weight of 1-[3'-(chloro)-phenyl]-4-[methyl]-5-[hydroxy]-7,8-di-[methoxy]-5H-2,3-benzodiazepine contamination of formula III are dissolved in it under boiling. When the dissolution is complete, the reaction mixture is cooled to 0 to 5°C and stirred at the same temperature for 4 hours. The separated crystals are filtered off, washed with isopropanol of 0 to 5°C and with distilled water to neutral and dried to constant weight at 40°C. Thus 4.6 g of white crystalline product are obtained which are recrystallized from a 12-fold volume of isopropanol related to the weight of the product to yield 4.3 g (86.0%) of the desired compound containing - according to HPLC examinations - 0.22% by weight of 1-[3'-(chloro)-phenyl]-4-[methyl]-5-[hydroxy]-7,8-di-[methoxy]-5H-2,3-benzodiazepine contamination of formula III.
M.p.: 167.5 to 168.5°C.

### Examination of the possible contaminations of 1-[3'-(chloro)-phenyl]-4-[methyl]-7,8-di-[methoxy]-5H-2,3-benzodiazepine carried out on the products of Examples 1 to 6

The possible contaminations of the compound of formula I are separated by HPLC using a Nucleosil® C₁₈ 10 µm (4 x 250 mm) column with a moving phase of 60 : 40 (by volume) mixture of acetonitrile and a 0.1 M acetate buffer (pH=4). The detection is carried out at 230 nm using an UV detector. The chromatograms are recorded on a Recording Integrator.

In the following Table the relative retention time and relative signal intensity of the possible contaminations of the compound of formula I measured at 230 nm and related to the peak of the main product (I) are shown.

**Table**

| Compound | Relative retention time | Relative signal intensity |
|---|---|---|
| 1-[3'-(Chloro)-phenyl]-4-[methyl]-5-[hydroxy]-7,8-di-[methoxy]-5H-2,3-benzodiazepine (III) | 0.57 | 0.94 |
| 2-(Acetonyl)-3'-(chloro)-4,5-di-(methoxy)-benzophenone (II) | 1.1 | 0.63 |
| 1-[3'-(Chloro)-phenyl]-4-[methyl]-8-[methoxy]-5H-2,3-benzodiazepine (IV) | 1.58 | 1.05 |
| 1-[3'-(Chloro)-phenyl]-3-[methyl]-6,7-di-[methoxy]-isoquinoline (V) | 1.46 | 1.37 |

## Claims

1. A process for the preparation of 1-[3'-(chloro)-phenyl]-4-[methyl]-7,8-di-[methoxy]-5H-2,3-benzodiazepine of formula 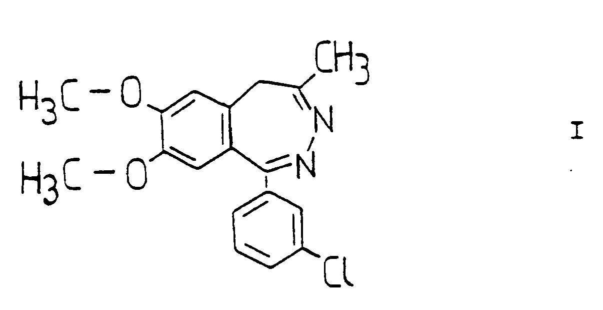 the total contamination content thereof not exceeding 0,3% by weight - according to high liquid pressure chromatographic examinations - by reacting 1 mole of 2-(acetonyl)-3'-(chloro)-4,5-di-(methoxy)-benzophenone of formula 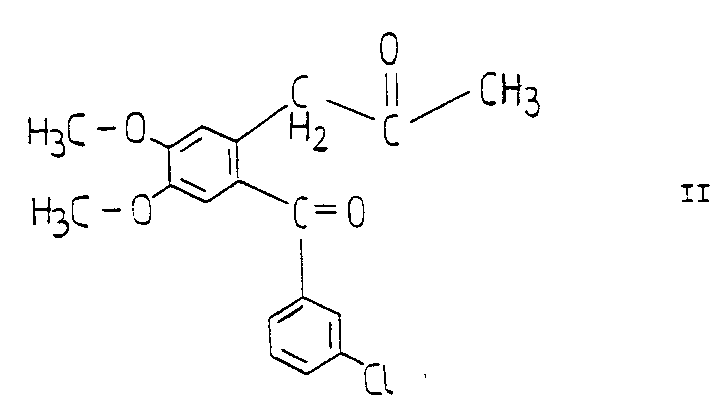 with 3 to 7 moles of hydrazine hydrate in an organic solvent or in a mixture of organic solvents, at a temperature from 15°C to 85°C, and subsequently recrystallizing the crude product from an aliphatic alcohol containing 1 to 5 carbon atom(s), characterized in that the reaction of the 2-(acetonyl)-3'-(chloro)-4,5-di-(methoxy)-benzophenone with the hydrazine hydrate is carried out in the absence of air oxygen.

2. A process as claimed in claim 1, characterized by carrying it out in the presence of water.

3. A process as claimed in claim 1 or 2, characterized by using as solvent(s) 1 or more monovalent or polyvalent aliphatic alcohol(s) comprising 1 to 5 carbon atom(s), dimethylformamide, dimethyl sulfoxide, 1 or more chlorinated hydrocarbon(s), peroxide-free dioxane or tetrahydrofuran or mixtures thereof or - in case of water-miscible solvents - a mixture of these solvents with water.

4. A process as claimed in claims 1 to 3, characterized by carrying out the reaction under an atmosphere of nitrogen and/or a noble gas.

5. A process for preparing purified 1-[3'-(chloro)-phenyl]-4-[methyl]-7,8-di-[methoxy]-5H-2,3-benzodiazepine of formula I from such containing contamination(s) of formula 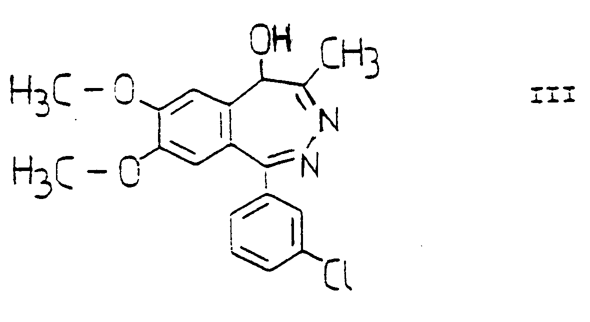 and optionally of formula 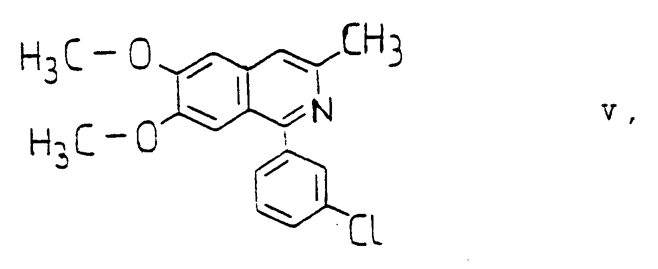 optionally prepared according to claims 1 to 4, characterized in that first the contaminated 1-[3'-(chloro)-phenyl]-4-[methyl]-7,8-di-[methoxy]-5H-2,3-benzodiazepine of formula I is treated with a solvent and an alkali hydroxide, alkali carbonate or alkali alcoholate the solvent being such in which at least a 0.005 mole/l solution from the said alkali compounds can be prepared and thereafter the compound 1-[3'-(chloro)-phenyl]-4-[methyl]-7,8-di-[methoxy]-5H-2,3-benzodiazepine of formula I is crystallized from the obtained solution.

6. A process as claimed in claim 5, characterized by recrystallizing from an aliphatic alcohol containing 1 to 5 carbon atom(s) the crystallized 1-[3'-(chloro)-phenyl]-4-[methyl]-7,8-di-[methoxy]-5H-2,3-benzodiazepine of formula I obtained.

7. A process as claimed in claim 5 or 6, characterized by using as solvent a straight or branched chained monovalent or polyvalent aliphatic alcohol containing 1 to 5 carbon atom(s) or mixtures thereof.

8. A process as claimed in claims 5 to 7, characterized by carrying out the treatment with the alkali hydroxide, alkali carbonate or alkali alcoholate at the boiling point of the solvent.

## Patentansprüche

1. Verfahren für die Herstellung von 1-[3'-(Chlor)-phenyl]-4-[methyl]-7,8-di-[methoxy]-5H-2,3-benzodiazepin der Formel 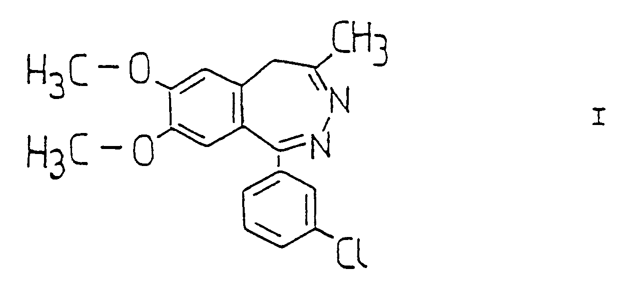 wobei der Gesamtgehalt an Verunreinigungen desselben 0,3 Gew.-% nicht übersteigt, nach Untersuchungen mittels Hochdruckflüssigchromatographie, indem 1 Mol 2-(Acetonyl)-3'-(chlor)-4,5-di-(methoxy)-benzophenon der Formel 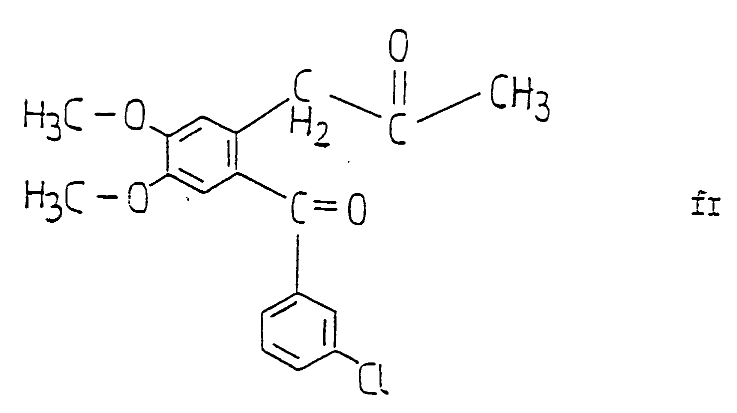 mit 3 bis 7 Mol Hydrazinhydrat in einem organischen Lösungsmittel oder in einer Mischung von organischen Lösungsmitteln bei einer Temperatur von 15°C bis 85°C umgesetzt und anschließend das Rohprodukt aus einem aliphatischen Alkohol mit 1 bis 5 Kohlenstoffatomen umkristallisiert wird, **dadurch gekennzeichnet**, daß die Umsetzung von 2-(Acetonyl)-3'-(chlor)-4,5-di-(methoxy)-benzophenon mit dem Hydrazinhydrat in Abwesenheit von Luftsauerstoff durchgeführt wird.

2. Verfahren nach Anspruch 1, **gekennzeichnet durch** die Durchführung in Gegenwart von Wasser.

3. Verfahren nach Anspruch 1 oder 2, **gekennzeichnet durch** die Verwendung als Lösungsmittel von 1 oder mehreren einwertigen oder mehrwertigen aliphatischen Alkoholen mit 1 bis 5 Kohlenstoffatomen, Dimethylformamid, Dimethylsulfoxid, 1 oder mehreren chlorierten Kohlenwasserstoffen, peroxidfreiem Dioxan oder Tetrahydrofuran oder Mischungen davon oder - im Falle von wassermischbaren Lösungsmitteln - einer Mischung dieser Lösungsmittel mit Wasser.

4. Verfahren gemäß den Ansprüchen 1 bis 3, **gekennzeichnet durch** die Durchführung der Reaktion unter einer Atmosphäre von Stickstoff und/oder eines Edelgases.

5. Verfahren zur Herstellung von gereinigtem 1-[3'-(Chlor)-phenyl]-4-[methyl]-7,8-di-[methoxy]-5H-2,3-benzodiazepin der Formel I, gereinigt von der enthaltenden Verunreinigung der Formel 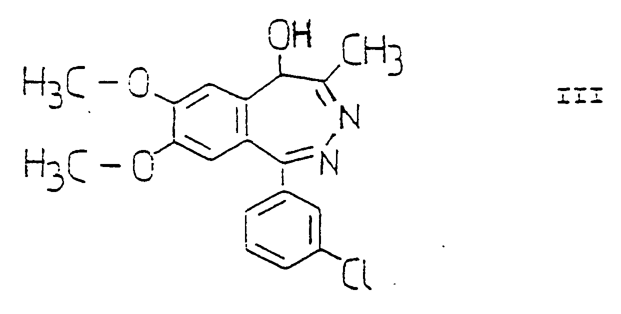 und gegebenenfalls der Formel 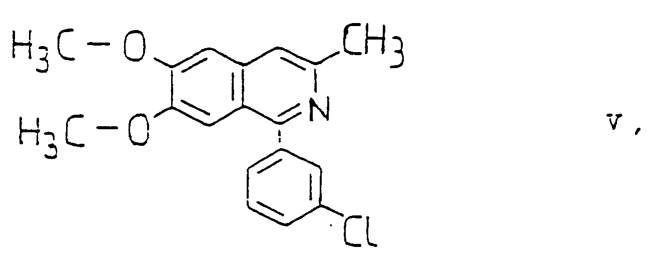 gegebenenfalls hergestellt gemäß den Ansprüchen 1 bis 4, **dadurch gekennzeichnet**, daß zuerst das verunreinigte 1-[3'-(Chlor)-phenyl]-4-[methyl]-7,8-di-[methoxy]-5H-2,3-benzodiazepin der Formel I mit einem Lösungsmittel und einem Alkalihydroxid, Alkalicarbonat oder einem Alkalialkoholat behandelt wird, wobei das Lösungsmittel ein solches ist, in dem mindestens eine Lösung von 0,005 Mol/l der Alkaliverbindungen hergestellt werden kann und anschließend die Verbindung 1-[3'-(Chlor)-phenyl]-4-[methyl]-7,8-di-[methoxy]-5H-2,3-benzodiazepin der Formel I aus der erhaltenen Lösung auskristallisiert wird.

6. Verfahren nach Anspruch 5, **dadurch gekennzeichnet**, daß das erhaltene kristallisierte 1-[3'-(Chlor)-phenyl]-4-[methyl]-7,8-di-[methoxy]-5H-2,3-benzodiazepin der Formel I aus einem aliphatischen Alkohol mit 1 bis 5 Kohlenstoffatomen umkristallisiert wird.

7. Verfahren nach Anspruch 5 oder 6, **gekennzeichnet durch** die Verwendung als Lösungsmittel eines geradkettigen oder verzweigtkettigen einwertigen oder mehrwertigen aliphatischen Alkohols mit 1 bis 5 Kohlenstoffatomen oder Mischungen davon.

8. Verfahren nach den Ansprüchen 5 bis 7, gekennzeichnet durch die Durchführung der Behandlung mit dem Alkalihydroxid, Alkalicarbonat oder Alkalialkoholat beim Siedepunkt des Lösungsmittels.

## Revendications

1. Un procédé de préparation de 1-[3'-(chloro)-phényl]-4-[méthyl]-7,8-di-[méthoxy]-5H-2,3-benzodiazépine représenté par la formule 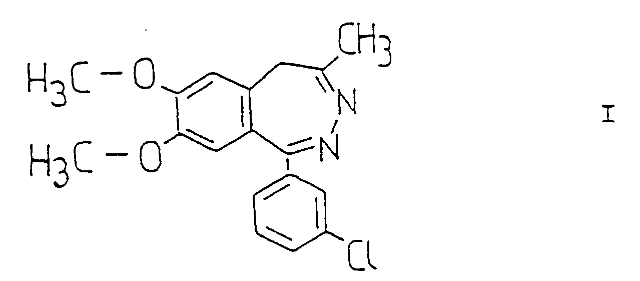 dont la teneur totale en contaminant - selon les analyses réalisées par chromatographie liquide haute pression - n'excède pas 0,3% en poids, ce procédé étant mis en oeuvre par réaction d'une mole de 2-(acétonyl)-3'-(chloro)-4,5-di-(méthoxy)-benzophénone de formule 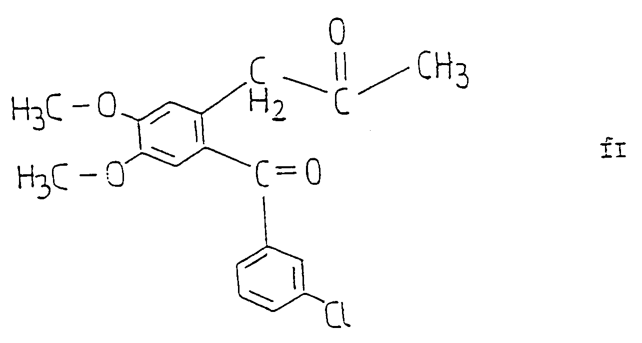 avec 3 à 7 moles d'hydrazine hydratée dans un solvant organique ou un mélange de solvants organiques, à une température de 15 à 85°C, et par recristallisation du produit brut à partir d'un alcool aliphatique contenant 1 à 5 atomes de carbone, caractérisé en ce que la réaction de la 2-(acétonyl)-3'-(chloro)-4,5-di-(méthoxy)-benzophénone avec l'hydrazine hydratée est mise en oeuvre en l'absence d'oxygène de l'air.

2. Procédé selon la revendication 1, caractérisé en ce qu'il est mis en oeuvre en présence d'eau.

3. Procédé selon la revendication 1 ou 2, caractérisé en ce que l'on utilise en tant que solvant(s) un ou de plusieurs alcools aliphatiques monohydriques ou polyhydriques comprenant 1 à 5 atomes de carbone, diméthylformamide, diméthyl sulfoxyde, un ou plusieurs hydrocarbures chlorés, tétrahydrofurane ou dioxane exempts de péroxyde ou leurs mélanges, ou - dans le cas de solvants miscibles à l'eau - un mélange de ces solvants avec de l'eau.

4. Procédé selon les revendications 1 à 3, caractérisé en ce que la réaction est mise en oeuvre sous une atmosphère d'un gaz rare et/ou d'azote.

5. Procédé de préparation de 1-[3'-(chloro)-phényl]-4-[méthyl]-7,8-di-[méthoxy]-5H-2,3-benzodiazépine purifiée à partir d'un tel composé de formule I contenant des contaminants, représenté par la formule I 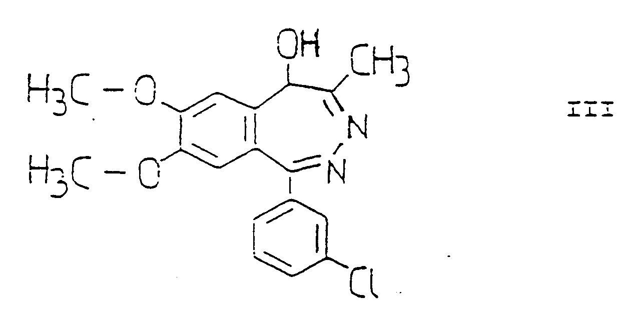 ou, éventuellement, par la formule V 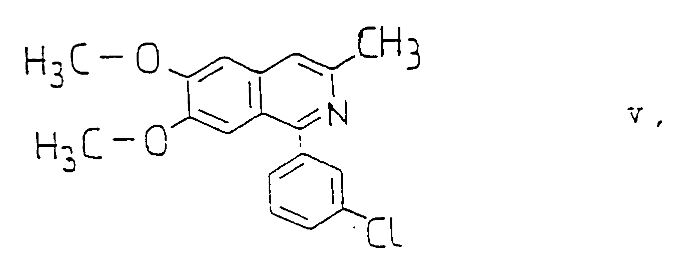 éventuellement préparé selon les revendications 1 à 4, caractérisé en ce que dans un premier temps, la 1-[3'-(chloro)-phényl]-4-[méthyl]-7,8-di-[méthoxy]-5H-2,3- benzodiazépine contaminée de formule I est traitée avec un solvant et un hydroxyde alcalin, carbonate alcalin ou alcoolate alcalin, le solvant étant un solvant dans lequel au moins à 0,005 mole/l desdits composés alcalins peut être préparéet ensuite la 1-[3'-(chloro)-phényl]-4-[méthyl]-7,8-di-[méthoxy]-5H-2,3-benzodiazépine de formule I est cristallisée dans la solution obtenue.

6. Procédé selon la revendication 5, caractérisé en ce que l'on procède à une recristallisation dans un alcool aliphatique contenant 1 à 5 atomes de carbone de la 1-[3'-(chloro)-phényl]-4-[méthyl]-7,8-di-[méthoxy]-5H-2,3-benzodiazépine cristallisée de formule I obtenue.

7. Procédé selon la revendication 5 ou 6, caractérisé en ce qu'on utilise en tant que solvant un alcool aliphatique monohydrique ou polyhydrique à chaîne linéaire ou ramifiée contenant 1 à 5 atomes de carbone ou les mélanges en dérivant.

8. Procédé selon les revendications 5 à 7, caractérisé en ce que le traitement est mis en oeuvre avec un hydroxyde alcalin, carbonate alcalin ou alcool alcalin au point d'ébullition du solvant.
